# EUROPEAN PATENT APPLICATION

(11) **EP 4 122 416 A1**
(43) Date of publication of application: **25.01.2023**
(21) Application number: 21186888.0
(22) Date of filing: 21.07.2021
(51) Int. Cl.: A61B 34/20, A61B 5/06

(54) **ELECTROMAGNETIC TRACKING FOR PERCUTANEOUS DILATATIONAL TRACHEOSTOMY**

(71) Applicant: Technische Universität München, 80333 München (DE)
(72) Inventor: Krumpholz, Roman, 85604 Zorneding (DE); Wilhelm, Dirk, 80469 München (DE); Ostler, Daniel, 81371 München (DE); Ranft, Andreas, 82377 Penzberg (DE)
(74) Representative: Lucke, Andreas

(57) **Abstract**

The present invention relates to systems and methods for performing a puncturing operation with improved puncture precision. The invention refers to a monitoring system comprising a first magnetic sensor attachable to a puncturing instrument (20), a second magnetic sensor (14) supportable by an endoscopic instrument (30), a field generator (16) configured for generating a variable magnetic field, and a processing unit. The processing unit (18) determines a relative position of the first magnetic sensor with respect to the second magnetic sensor and generates positional data encoding such relative position. The invention further refers to a puncturing system (100) comprising such a monitoring system (10), to a related method of generating positional data, and to a related storage device.

## Description

### FIELD OF THE INVENTION

The present invention is in the field of medical technology and relates in particular to systems and methods for performing a percutaneous dilatational tracheostomy with improved puncture precision.

### BACKGROUND OF THE INVENTION

A tracheostomy is a surgical procedure to create a direct connection between the exterior of the body of a patient and the interior of the trachea of the patient through an incision cut. Such connection bypasses the upper airways of the patient and can replace an endotracheal tube. In critically ill patients, the connection created during the tracheostomy can be used for providing mechanical ventilation. In Germany, around 50.000 tracheostomies are performed per year.

There are two basic techniques for performing a tracheostomy: the open surgical tracheostomy (OST) and the percutaneous dilatational tracheostomy (PDT). One major advantage of PDT is that it can be performed by an intensivist at the patient's bedside, while an OST must be performed in an operating room. This significantly decreases costs, time and manpower requirements. Further, PDT entails a lower risk of infection as compared to OST.

A PDT is commonly performed under general anaesthesia with the patient's head reclined. The surgeon or intensivist tries to identify the trachea by palpation, in particular the interspace between the second and the third tracheal rings (counted from the larynx), which typically is the targeted puncture location.

A crucial aspect for the success of a PDT is the correct choice of the point and orientation at which the skin is punctured. Correctly determining the entry point and trajectory of the puncturing instrument is not always straightforward, for example when excessive soft tissue of the neck prevents a clear palpatory localisation of the trachea, when a rigid cervical spine affects the exposure/palpability of the trachea, or when the course of the trachea is off-centre. In such situations, it is common to choose the OST approach rather than the PDT approach at the price of accepting increased costs, time, medical staff, and increased risk of infection.

A PDT can be endoscopically monitored using a bronchoscope introduced into the trachea of the patient through the endotracheal tube in order to provide visual monitoring from within the trachea. However, the puncture must be done blindly until the needle of the puncturing instrument is endoscopically visible. Thus, while endoscopic monitoring can generally be used for assessing the correct entry of the puncturing instrument into the tracheal lumen, it cannot not prevent the puncturing instrument from being misdirected and/or mispositioned, which can lead to undesired injuries of surrounding blood vessels such as the brachiocephalic and carotid artery, the thyroid gland, the pleura, the trachea itself or to damage of the bronchoscope itself.

Alternative methods for improving the puncturing precision are based on providing internal transillumination of the tracheal region using a light emitting endoscope (diaphanoscopy) or on the use of ultrasound imaging for exploring the tracheal region before puncturing. However, these techniques cannot per se solve the aforementioned disadvantages: light does not pass through thick soft tissue, and with a short, immobile cervical spine there is often not enough space for properly obtaining ultrasound images. Taken together, none of these techniques reliably overcomes the risks and disadvantages of the aforementioned situations where PDT is usually contraindicated.

Similar situations apply to other surgical interventions upon the body of a patient requiring a surgical puncturing operation for creating an incision or access to the body of the patient.

Therefore, there is room for technical improvement in the field of surgical puncturing operations.

### SUMMARY OF THE INVENTION

The present invention aims at overcoming the aforementioned disadvantages of the prior art, in particular by providing a solution to the problem of correctly determining the puncturing location and/or orientation for performing a surgical puncturing operation and of providing real-time guidance during the puncturing operation. This problem is solved by a monitoring system according to claim 1, by a puncturing system according to claim 10, by a method of generating positional data for monitoring a surgical puncturing operation according to claim 11, and by a corresponding memory device according to claim 15. Preferred embodiments of the invention are defined in the appended dependent claims.

The invention is related to an exemplary method of performing a surgical puncturing operation that is useful for the understanding of the invention. Such method is described below.

A first aspect of the invention refers to a monitoring system for monitoring a surgical puncturing operation, in particular for a percutaneous dilatational tracheostomy (PDT). A "surgical puncturing operation" may refer herein to any surgical operation used for puncturing the body of a patient, in particular a hollow organ, tissue or body part, during which a puncturing instrument may be used for puncturing the body of a patient and may further be introduced into the body of the patient. The "surgical puncturing operation" may for example be a percutaneous puncture of a trachea, in particular as part of a PDT: The system comprises a first magnetic sensor, a second magnetic sensor, a field generator and a processing unit.

The first magnetic sensor is arrangeable on and/or attachable to a puncturing instrument configured for performing a surgical puncturing operation, in particular a percutaneous puncture of a trachea. The puncturing instrument may for example comprise a syringe and an incision needle or cannula, wherein the incision needle or cannula maybe at least in part movably received within the syringe, such that the incision needle or cannula, in particular an incision tip thereof, can be selectively moved with respect to the syringe to protrude distally from the syringe for performing the puncturing operation, in particular by actuating the piston of the syringe. The first magnetic sensor may be configured for being arranged on and/or attached to the syringe, for example at a distal end of the syringe, such that the position of the first magnetic sensor does not vary with respect to the syringe when the incision needle or cannula is moved with respect to the syringe.

The second magnetic sensor is supportable by an endoscopic instrument, in particular an endoscopic instrument configured for providing endoscopic monitoring of a puncturing operation performed by the puncturing instrument from within the body of the patient. The endoscopic instrument may for example be a bronchoscope configured for providing endoscopic monitoring of a PDT from within the trachea of the patient. "Supportable" refers herein to the fact that the second magnetic sensor is configured for being supported by the endoscopic instrument. In other words, the monitoring system is configured such that the second magnetic sensor can be held in a position by the endoscopic instrument, in particular by a part of the endoscopic instrument insertable into the body of a patient in a vicinity of a target puncturing location.

The second magnetic sensor may be arrangeable on and/or attachable to the endoscopic instrument, in particular to said part of the endoscopic instrument insertable into the body of a patient in a vicinity of a target puncturing location. The second magnetic sensor may in particular be configured for being arranged at or in front of a visualisation tip of the endoscopic instrument, i.e. at or in front of a distal end of the endoscopic instrument that is closest to the region of the patient being monitored by the endoscopic instrument.

The second magnetic sensor may be insertable through the endoscopic instrument. Thus, the second magnetic sensor may be insertable into the body of the patient into a proximity of the target puncturing location through the endoscopic instrument.

An endoscopic instrument may comprise an optical channel and a working channel, arranged at least in part in parallel to the optical channel or lumen. The optical channel may be configured for establishing an optical link between a visualisation tip of the endoscopic instrument and an observation end of the endoscopic instrument, opposite the visualisation tip, in order to allow a visualisation of a body region arranged in front of the visualisation tip from the observation end through the optical channel of the endoscopic instrument. The optical channel may for example comprise an optical fiber received therein, extending from the visualisation tip to the observation end. The working channel may allow accessing said internal region of the body that is visible through the optical channel with other instruments that may be inserted through the working channel, for example a wire or thread, a stylet or the like.

The working channel of an endoscopic instrument can be used for inserting therethrough the second magnetic sensor, for example using a wire or thread attached to the second magnetic sensor. For example, the endoscopic instrument can first be inserted into the body of a patient and arranged such that the visualisation tip of the optical channel is in front of the target puncturing location (i.e. such that the target puncturing location can be seen from the observation end through the optical channel of the endoscopic instrument). Then, a working channel of the endoscopic instrument can be used for inserting the second magnetic sensor therethrough in order to arrange it next to the target puncturing location. This can be done, for example, by advancing the second magnetic sensor through the working channel using a wire attached to the second magnetic sensor until the second magnetic sensor is visible through the optical channel. The second magnetic sensor is then supported by the endoscopic instrument via said wire.

The field generator is configured for generating a variable magnetic field. "Variable magnetic field" may refer herein in particular to one or more magnetic fields with time-varying magnetic field strengths, wherein the magnetic field strengths may in particular vary periodically with a preset frequency or period. The field generator may for example be configured for generating an electromagnetic field with a continuously varying magnetic field strength and a given frequency, for instance 800 Hz.

The first magnetic sensor is configured for generating first position data in response to the magnetic field generated by the field generator, wherein the first position data is indicative of a position of the first magnetic sensor, in particular with respect to the field generator. The second magnetic sensor is configured for generating second position data in response to the magnetic field generated by the field generator, wherein the second position data is indicative of a position of the second magnetic sensor, in particular with respect to the field generator. The first and second position data may refer herein to any kind of information related to measurable parameters of the magnetic field generated by the field generator and/or by electric currents induced by said magnetic field suitable for determining the position of the first magnetic sensor and the second magnetic sensor, respectively.

The first magnetic sensor and/or the second magnetic sensor may be or may comprise a respective electromagnetic sensor. The first magnetic sensor and/or the second magnetic sensor may be or may comprise inductive sensors comprising one or more induction coils. The first and second magnetic sensors are responsive to the magnetic field generated by the field generator and may in particular correspond to magnetic sensors of an electromagnetic tracking system, whereas the field generator may correspond to the field generator of an electromagnetic tracking system. For example, the field generator may correspond to a "magnetic transmitter" configured for outputting magnetic fields as described in international patent application WO 2020/146354 A1. The first and second magnetic sensors may correspond to "probes" configured for determining locations and/or orientations in a magnetic field as described in European patent application EP 1272862 Ai, for example based on the physical model described therein for determining values of magnetic fluxes measured by the probes and/or on the iterative processes described therein for determining a probe's location and orientation.

The processing unit of the monitoring system of the invention is configured for determining a relative position of the first magnetic sensor with respect to the second magnetic sensor based on the first and second position data. Thus, the combination of the first and second magnetic sensors with the field generator and the processing unit, in particular a module or subunit thereof that may be responsible for determining a position of the first magnetic sensor in absolute terms and a position of the second magnetic sensor in absolute terms, may be regarded as an electromagnetic tracking system allowing to determine the position of each of the first and second magnetic sensors in ways that are accessible to a person skilled in the art of electromagnetic tracking, for example from the aforementioned publications WO 2020/146354 A1 and EP 1272862 A1.

However, rather than focusing on determining the position of each of the first and second magnetic sensors in absolute terms, the processing unit of the monitoring system of the invention is specifically configured for determining a relative position of both (first and second) magnetic sensors.

The processing unit may be functionally connected with the first magnetic sensor, the second magnetic sensor and/or the field generator. From the first magnetic sensor and the second magnetic sensor, the processing unit may obtain the first position data and the second position data, respectively. From the field generator, the processing unit may obtain information about the variable magnetic fields generated by the field generator. The processing unit may be configured for determining the relative position of the first magnetic sensor with respect to the second magnetic sensor based on the first and second position data and based on information about the variable magnetic field obtained from the field generator. The processing unit may be functionally connected with the field generator by means of a wired connection, although a wireless connection can also be used in some embodiments.

According to the invention, the processing unit is further configured for generating positional data encoding the determined relative position of the first magnetic sensor with respect to the second magnetic sensor. Optionally, the processing unit may further be configured for outputting the generated positional data to a graphical representation unit, preferably in real time.

The positional data generated by the processing unit can be transmitted to a graphical representation unit for providing a graphical representation of the relative position of the first magnetic sensor and/or of the puncturing instrument with respect to the second magnetic sensor and/or of the endoscopic instrument for assisting a surgeon or intensivist performing the surgical puncturing operation, in particular in real time. For instance, the position of the first magnetic sensor and/or of the puncturing instrument to which the first magnetic sensor may be attached, may be represented relative to the position of the second magnetic sensor, which may be indicative of the position of the target puncturing location. This may allow the intervening surgeon or intensivist to track the relative position of the first magnetic sensor and/or of the puncturing instrument with respect to the target puncturing location in order to reach the target puncturing location with high accuracy.

Thus, the monitoring system of the invention is specifically configured for providing improved positional precision for performing a surgical puncturing operation, in particular a puncturing of the trachea within the context of a PDT. The first magnetic sensor can be attached to and/or arranged on a puncturing instrument used for performing the surgical puncturing operation and the second magnetic sensor can be supported by an endoscopic instrument used for providing endoscopic monitoring of the puncturing operation from within the body of the patient.

For example, the first magnetic sensor can be attached to a syringe configured for performing a PDT, with an incision needle or cannula movably received within the syringe, and the second magnetic sensor can be arranged in front of the visualisation tip of a bronchoscope used for monitoring the puncturing operation from within the trachea, for example supported by a wire received within a working channel of the bronchoscope. Then, the positional data generated by the processing unit can be used by a user of the monitoring system of the invention for monitoring a position of the puncturing instrument with respect to a target puncturing location, in order to select the puncturing location on the skin of the patient with high spatial precision. The target puncturing location may have been previously determined from within the trachea by correspondingly positioning the second magnetic sensor, supported by the endoscopic instrument/bronchoscope, within the trachea, for example next to the target puncturing location.

The processing unit may be configured for taking into account the arrangement of the first magnetic sensor with respect to the puncturing instrument and/or the arrangement of the second magnetic sensor with respect to the endoscopic instrument. For example, when the puncturing instrument is a syringe with an incision needle or cannula movably received therein, the first magnetic sensor may be attached to the syringe and the processing unit may be configured for taking into account the distance between the position of the first magnetic sensor and the tip of the incision needle, in particular the tip of the incision needle when the incision needle is maximally protruding from the syringe for performing the puncturing operation. Such distance may be encoded in the positional data as well. This allows generating the positional data such that a correct graphical representation of the (potential) position of the tip of the incision needle with respect to the target puncturing location marked by the second magnetic sensor can be graphically represented without having to arrange the first magnetic sensor on the incision needle, which would difficult the puncturing operation. Instead, the first magnetic sensor can be arranged on the syringe itself, which preferably does not penetrate into the body of the patient during the puncturing operation.

For example, the second magnetic sensor may be positioned within the trachea, supported by the endoscopic instrument, such that the second magnetic sensor is arranged at the target puncturing location, in particular at the frontal tracheal wall, at a given distance, for example 2 to 10 mm, from the target puncturing location on the skin of the patient in the ventral-dorsal direction. Then, the processing unit can be configured for generating the positional data taking into account a distance in the ventral-dorsal direction between the first magnetic sensor (arranged on the syringe containing the incision needle) and the second magnetic sensor corresponding to said given distance plus an additional distance corresponding to a distance between the first magnetic sensor and the tip of the movable incision needle when the incision needle is maximally protruding from the syringe, for example plus 5 mm. The positional data is hence usable for representing the precise (potential) relative position of the incision needle (when completely protruding from the syringe) with respect to the second magnetic sensor, i.e. with respect to the target puncturing location. The processing unit maybe likewise configured for taking into account any offset of the first magnetic sensor with respect to the second magnetic sensor in any direction, for example in the cranial-caudal direction.

Thus, a user of the monitoring system of the invention, who may in particular be a surgeon or an intensivist, can reliably reach the desired target puncturing location, for example a location of the skin of the patient arranged directly above the anterior tracheal wall. The monitoring system of the invention generates positional data that can be used for assisting the surgeon or intensivist during the surgical puncturing operation, in particular both before performing the puncturing operation, i.e. for determining the puncture location on the skin of the patient, and while the PDT is being finalised, for example while an incision needle is advanced into the trachea. The positional data generated by the monitoring system of the invention can be used for providing real-time guidance throughout the preparation and execution of a puncturing operation, thereby preventing collateral damages to surrounding tissue or to the endoscopic instrument itself even under difficult anatomical conditions. In particular, the monitoring system of the invention allows performing a PDT with improved positional precision even with restricted neck mobility as well as with a short and/or thick neck.

Compared to the use of ultrasound imaging, the monitoring system of the present invention offers the additional advantage of not obstructing the access to the front of the neck of the patient, as opposed to the use of an ultrasound scanner, the transducer of which is often a voluminous instrument that reduces the accessibility by the surgeon or intensivist to the puncturing location.

According to preferred embodiments, the positional data may be based on a coordinate system having the position of the second magnetic sensor as an origin of coordinates. Having the positional data in such format can allow a graphical representation unit to correspondingly represent the positions of the first magnetic sensor and/or of the puncturing instrument and of the second magnetic sensor and/or of the endoscopic instrument from a perspective centered in the second magnetic sensor. Thereby, the positioning of the puncturing instrument can be easily adjusted, in particular while the endoscopic instrument and/or the second magnetic sensor is/are left at rest. The processing unit may be configured for generating the positional data based on the aforementioned coordinate system, such that the positional data is specifically configured and/or formatted for allowing a graphical representation of the position of the first magnetic sensor and/or the puncturing instrument with respect to the second magnetic sensor.

In preferred embodiments of the invention, the monitoring system may further comprise a graphical representation unit configured for providing a graphical representation of the relative position of the first magnetic sensor with respect to the second magnetic sensor, preferably in real time. The graphical representation may in particular be based on the positional data generated by the processing unit. The graphical representation unit may for example be or comprise a graphic card, a screen or the like and may allow an operator of the monitoring system, in particular a surgeon or intensivist, to directly visualise the relative position of the first magnetic sensor with respect to the second magnetic sensor, preferably in real time, while performing the surgical puncturing operation. The graphical representation may be a graphical representation of the relative position of the first and second magnetic sensors, and/or of the relative position of the puncturing instrument with respect to the endoscopic instrument or to a reference position.

In addition to or instead of being configured for generating the positional data as described above, the processing unit may be configured for generating an indication signal, which may for example be used for triggering an acoustic signal and/or a visual signal, when the determined relative position corresponds to a target relative position within a predefined tolerance. The indication signal may be a communication signal, for example an electronic signal, usable for triggering an acoustic signal and/or a visual signal. For instance, the indication signal may trigger a colour indicator in a graphical representation of the positional data generated by the processing unit when the relative position corresponds to the target relative position within the predefined tolerance.

This means that the processing unit may be configured for generating the indication signal when a position of the first magnetic sensor relative to the second magnetic sensor is determined to correspond to a predetermined spatial relationship between the first magnetic sensor and the second magnetic sensor, i.e. the "target relative position", which may for example indicate that the position of the first magnetic sensor corresponds to (i.e. overlaps with) the position of the second magnetic sensor within the predefined tolerance and/or that a distance between the first magnetic sensor and the second magnetic sensor corresponds to a predefined distance within the predefined tolerance, for example 1.0 ± 0.1 mm. The predefined distance may be from 0.1 mm to 10 mm, preferably from 0.5 mm to 5 mm. The predefined tolerance may be from 1 mm to 2 mm.

As previously mentioned, when the first magnetic sensor is attached to a syringe in which an incision needle or cannula is movably received, the processing unit may be configured for taking into account distance between the position of the first magnetic sensor and the tip of the incision needle, in particular the tip of the incision needle when the incision needle is maximally protruding from the syringe for performing the puncturing operation. This also applies to the generation of indication signal.

For example, if the endoscopic instrument is positioned within the trachea such that the second magnetic sensor is arranged dorsally from the target puncturing location at a given distance, for example 2 to 10 mm, the processing unit can be configured for generating the indication signal when the determined relative position is indicative of a distance between the first magnetic sensor (arranged on the syringe containing the incision needle) and the second magnetic sensor in the ventral-dorsal direction corresponding to said given distance plus an additional distance corresponding to a distance between the first magnetic sensor and the tip of the movable incision needle when the incision needle is maximally protruding from the syringe, for example plus 5 mm. The signal can hence indicate the precise position of the catheter at which the incision needle should be moved with respect to the syringe for puncturing the trachea precisely at the target puncturing location marked by the second magnetic sensor.

According to preferred embodiments, the first magnetic sensor may further be configured for generating first orientation data in response to the magnetic field generated by the field generator. The first orientation data are indicative of an orientation of the first magnetic sensor and/or of a puncturing instrument to which the first magnetic sensor may be attached, preferably with respect to the second magnetic sensor. The first orientation data may refer herein to any kind of information related to measurable parameters of the magnetic field generated by the field generator and/or by electric currents induced by said magnetic field suitable for determining the orientation of the first magnetic sensor and/or of a puncturing instrument to which the first magnetic sensor may be attached. The processing unit may then be further configured for determining an orientation of the first magnetic sensor and/or of the puncturing instrument, preferably with respect to the second magnetic sensor, based on the first orientation data. The processing unit may thereby obtain and process not only information about the position of the first magnetic sensor and/or the puncturing instrument, but also information about the orientation thereof. Such information, which may be encoded in the positional data generated by the processing unit and/or may be used for generating the indication signal described above, may be used for allowing an operator of the monitoring system of the invention to identify not only the correct target puncturing location but also the corresponding correct orientation of the puncturing instrument for performing the puncturing operation with a desired puncturing orientation.

In some embodiments, the processing unit may further be configured for generating orientational data encoding the orientation of the first magnetic sensor and/or of the puncturing instrument, preferably with respect to the second magnetic sensor. Optionally, the processing unit may be configured for outputting the generated orientational data to a graphical representation unit, preferably in real time. The orientational data generated by the processing unit can be transmitted to a graphical representation unit for providing a graphical representation of the orientation of the first magnetic sensor and/or of the puncturing instrument, in particular in combination with a graphical representation of the relative position of the first magnetic sensor and/or of the puncturing instrument with respect to the second magnetic sensor and/or of the endoscopic instrument for assisting the surgeon or intensivist performing the surgical puncturing operation.

In preferred embodiments, the monitoring system may further comprise a second graphical representation unit configured for providing a graphical representation of the orientation of the first magnetic sensor and/or of the puncturing instrument, preferably with respect to the second magnetic sensor, preferably in real time. The graphical representation by the second graphical representation unit may in particular be based on the orientational data generated by the processing unit and may in particular be a graphical representation of the first magnetic sensor and/or of a puncturing instrument to which the first magnetic sensor maybe attached. The (first) graphical representation unit mentioned above and the second graphical representation unit may be the same graphical representation unit, which may be configured for graphically representing the orientation of the first magnetic sensor and/or of the puncturing instrument, in particular in combination with a graphical representation of the relative position of the first magnetic sensor and/or of the puncturing instrument with respect to the second magnetic sensor and/or the endoscopic instrument for assisting the surgeon performing the surgical puncturing operation.

In preferred embodiments of the invention, the processing unit may be connected with the first magnetic sensor and/or with the second magnetic sensor via a wired connection. Thereby, stable communication of the first magnetic sensor and/or the second magnetic sensor with the processing unit can be guaranteed and protected from environmental disruptions. However, in other embodiments, the processing unit may be connected with the first magnetic sensor and/or with the second magnetic sensor via a wireless connection.

A wire used for providing a wired connection between the second magnetic sensor and the processing unit may be configured for allowing the endoscopic instrument to support the second magnetic sensor by being received within the endoscopic instrument, in particular within a working channel of the endoscopic instrument. Thus, the monitoring system according to the invention can comprise one or more wires configured for providing both structural support to the corresponding (second) magnetic sensor and a communication connection with the processing unit. The wire may for example be a metallic wire, possibly coated with an insulating coating.

In preferred embodiments of the invention, the processing system may further comprise an adapter for arranging the first magnetic sensor on a puncturing instrument at a fixed position and/or at a fixed orientation. The adapter can guarantee that the first magnetic sensor stays in a fixed position and/or orientation during the puncturing operation, thereby ensuring a sufficient degree of positional and/or orientational precision. If, as previously described, the puncturing instrument comprises a syringe and an incision needle or cannula movably received therein, the adapter may in particular be configured for arranging the first magnetic sensor on the syringe at said fixed position and/or said fixed orientation, for example at a distal end of the syringe closest to the puncturing location. This guarantees that a prediction of the processing unit of a potential position of the movable needle or catheter based on the position data provided by the first magnetic sensor is reliable. Preferably, the adapter may be configured for attaching the first magnetic sensor to the puncturing instrument such that a relative position and/or orientation between the first magnetic sensor and the incision tip of the incision needle or catheter, when the incision needle is maximally protruding from the syringe, is fixed.

In preferred embodiments of the invention, the field generator may be configured for generating the variable magnetic field having a continuously variable magnetic field strength.

In preferred embodiments of the invention, the field generator may be configured for generating the variable magnetic field having a frequency of 100 Hz to 10 kHz, preferably of 500 Hz to 1.5 kHz.

A second aspect of the invention refers to a puncturing system for performing a surgical puncturing operation, which may in particular be a PDT-system for performing a PDT. The puncturing system comprises a monitoring system according to any of the embodiments of the first aspect of the invention, a puncturing instrument and an endoscopic instrument. The puncturing instrument is configured for performing a surgical puncturing operation on the body of a patient. The first magnetic sensor of the monitoring system is arrangeable or arranged on the puncturing instrument, preferably on an incision tip thereof. As previously mentioned, the puncturing instrument may for example comprise a syringe and an incision needle or cannula movably received within the syringe.

The endoscopic instrument is configured for providing endoscopic monitoring of the surgical puncturing operation by the puncturing instrument, in particular from within the body of the patient. The second magnetic sensor of the monitoring system is supportable by the endoscopic instrument. For example, the second magnetic sensor of the monitoring system may be arranged on the endoscopic instrument and/or attached thereto, preferably at a visualisation tip thereof. Additionally or alternatively, the second magnetic sensor may be supported by the endoscopic instrument by being attached to a wire that is received within the endoscopic instrument, in particular within a working channel of the endoscopic instrument, while an optical channel of the endoscopic instrument provides visual access to an environment of the visualisation tip of the endoscopic instrument. Said wire may further provide a wired connection between the second magnetic sensor and the processing unit of the monitoring system. The endoscopic instrument may in particular be or may comprise a bronchoscope.

Preferably, the processing unit of the monitoring system may be configured for registering a position and/or an orientation of the puncturing instrument and/or of the endoscopic instrument by correspondingly taking into account an arrangement of the first magnetic sensor on the puncturing instrument and/or an arrangement of the second magnetic sensor with respect to the endoscopic instrument.

According to some embodiments, the processing unit of the monitoring system may be configured for determining a relative position of the puncturing instrument with respect to the second magnetic sensor based on the first and second position data (and/or from first and second orientation data). The processing unit may then be configured for generating the positional data encoding the determined relative position of the puncturing instrument with respect to the second magnetic sensor. For example, by taking into account a position and/or an orientation of the first magnetic sensor with respect to the puncturing instrument, the processing unit may be able to derive from the first position data (and/or from first orientation data), respectively, a corresponding position and/or orientation of the puncturing instrument with respect to the second magnetic sensor.

The puncturing system of the invention allows performing a puncturing operation, for example a puncturing of the trachea as part of a PDT, while monitoring the puncturing operation using the monitoring system, thereby providing increased positional precision and reduced risks of collateral damage due to wrong positioning and/or wrong orientation of the puncturing instrument.

A third aspect of the invention refers to a method of generating positional data for monitoring a surgical puncturing operation. The method, which may in particular be a computer implemented method, comprises:
- obtaining first measurement data corresponding to a position of a first magnetic sensor arranged on and/or attached to a puncturing instrument configured for performing a surgical puncturing operation on the body of a patient;
- obtaining second measurement data corresponding to a position of a second magnetic sensor supported by an endoscopic instrument configured for providing endoscopic monitoring of the surgical puncturing operation by the puncturing instrument from within the body of the patient, wherein the second magnetic sensor may be preferably attached to a wire received within the endoscopic instrument and/or passed therethrough.

The first and second measurement data are respectively obtained in response to a variable magnetic field to which the first and second magnetic sensors are exposed. The variable magnetic field may correspond to the magnetic field generated by the field generator according to any of the embodiments of the previously described aspects of the invention. The method further comprises:
- determining a relative position of the first magnetic sensor and/or of the puncturing instrument with respect to the second magnetic sensor and/or the endoscopic instrument based on the first and second measurement data; and
- generating positional data encoding the determined relative position.

Thus, the method according to the third aspect of the present invention results in the generation of positional data encoding the relative position of the first magnetic sensor and/or of the puncturing instrument with respect to the second magnetic sensor and/or the endoscopic instrument, which can then be used for assisting a surgeon or an intensivist when performing a surgical puncturing operation, in particular in real time, for example by graphically representing the generated positional data. The second magnetic sensor can be purposely arranged next to a target puncturing location, such that a relative position of the first magnetic sensor and/or of the puncturing instrument with respect to the target puncturing location can be derived from the generated positional data. The generated positional data can be transmitted to a graphical representation unit for graphical representation.

The method may be performed by the processing unit of a monitoring system according to the first aspect of the invention or by any processing unit in general, for example by a CPU.

In some preferred embodiments, the method may further comprise generating an indication signal when the determined relative position corresponds to a target relative position within a predefined tolerance. Such "target relative position" may identically correspond to the target relative position described above with respect to the first aspect of the invention.

According to some embodiments, the method may further comprise outputting the generated positional data to a graphical representation unit.

In preferred embodiments, the positional data may be generated based on a coordinate system having the position of the second magnetic sensor as an origin of coordinates.

According to some embodiments, the first measurement data may further correspond to an orientation of the first magnetic sensor. The method may then further comprise determining an orientation of the first sensor based on the first measurement data and generating orientational data encoding the determine orientation of the first sensor and/or of the puncturing instrument, preferably with respect to the second magnetic sensor.

Preferably, the method may further comprise outputting the generated orientational data to a graphical representation unit, in particular in combination with the aforementioned positional data.

A fourth aspect of the invention refers to a memory device storing executable instructions which, when executed by a processor, cause the processor to perform the method of any of the embodiments of the third aspect of the invention.

A method of performing a surgical puncturing operation, in particular for a PDT, may be performed using the puncturing system according to the second aspect of the invention. The method may comprise:
- positioning the second magnetic sensor of the puncturing system within the body of a patient at a relative position with respect to a target puncturing locationtarget puncturing location, wherein the second magnetic sensor is supported by the endoscopic instrument of the puncturing system, preferably by means of a wire, wherein the wire optionally connects the second magnetic sensor to the processing unit of the monitoring system;
- while generating, by the field generator of the monitoring system of the puncturing system, a variable magnetic field applied to the first and second magnetic sensors of the monitoring system of the puncturing system, positioning the puncturing instrument of the puncturing system above the body of the patient at a position at which the positional data generated by the processing unit of the monitoring system of the puncturing system encodes that a position of the first magnetic sensor and/or of the puncturing instrument corresponds to a position of the second magnetic sensor and/or of the target puncturing location within a predefined tolerance; and
- puncturing the body of the patient with the puncturing instrument at said position at which the positional data generated by the processing unit of the monitoring system of the puncturing system encodes that a position of the first magnetic sensor and/or of the puncturing instrument corresponds to a position of the second magnetic sensor and/or of the target puncturing location within the predefined tolerance.

In embodiments in which the processing unit of the puncturing system is configured for generating an indication signal when the relative position of the first magnetic sensor and/or of the puncturing instrument with respect to the second magnetic sensor corresponds to a target relative position within a predefined tolerance, in the previously described method, the instant in which the positional data encodes that the position of the first magnetic sensor and/or of the puncturing instrument corresponds to the position of the second magnetic sensor and/or of the target puncturing location within the predefined tolerance may correspond to the generation of the indication signal.

According to some embodiments, the first magnetic sensor of the monitoring system of the puncturing system may be configured for generating orientation data indicative of an orientation of the first magnetic sensor and/or of the puncturing instrument, preferably with respect to the second magnetic sensor. The processing unit may then be configured for determining an orientation of the first magnetic sensor and/or of the puncturing instrument, preferably with respect to the second magnetic sensor, based on the orientation data generated by the first magnetic sensor. The method may then further comprise, while generating, by the field generator of the monitoring system of the puncturing system, the variable magnetic field, orienting the puncturing instrument of the puncturing system with an orientation at which the orientation of the first magnetic sensor and/or of the puncturing instrument determined by the processing unit corresponds to a target orientation and puncturing the body of the patient at said position at which the positional data generated by the processing unit of the monitoring system of the puncturing system encodes that a position of the first magnetic sensor and/or of the puncturing instrument corresponds to a position of the second magnetic sensor and/or of the target puncturing location within the predefined tolerance with the puncturing instrument oriented with said target orientation.

Notably, while the use of electromagnetic tracking systems in medical technology has been known for some time, the present inventors found out a specific advantageous use of this technology when applied to chirurgical puncturing operations such as PDT, which can be exploited by means of the different aspects of the present invention.

### BRIEF DESCRIPTION OF THE FIGURES

- Fig. 1: shows a schematic illustration of a use of a puncturing system according to some embodiments of the invention.
- Fig. 2: shows a schematic zoomed-in view of Fig. 1 for illustrating the principles of the invention.
- Fig. 3: shows schematic illustrations of a graphical representation of positional data generated according to the invention.
- Fig. 4: shows schematic illustrations of a graphical representation of positional and orientational data generated according to the invention.
- Fig. 5: shows a schematic flow diagram illustrating a method of generating positional data for monitoring a surgical puncturing operation according to some embodiments of the invention.
- Fig. 6: shows a schematic flow diagram illustrating a method of performing a surgical puncturing operation using a puncturing system according to the invention.

### DETAILED DESCRIPTION OF PREFERRED EMBODIMENTS OF THE INVENTION

For the purposes of promoting an understanding of the principles of the invention, reference will now be made to specific preferred embodiments illustrated in the drawings, and specific language will be used to describe the same. It will nevertheless be understood that no limitation of the scope of the invention is thereby intended, such alterations and further modifications in the illustrated apparatus and such further applications of the principles of the invention as illustrated therein being contemplated as would normally occur now or in the future to someone skilled in the art to which the invention relates within the scope defined by the claims.

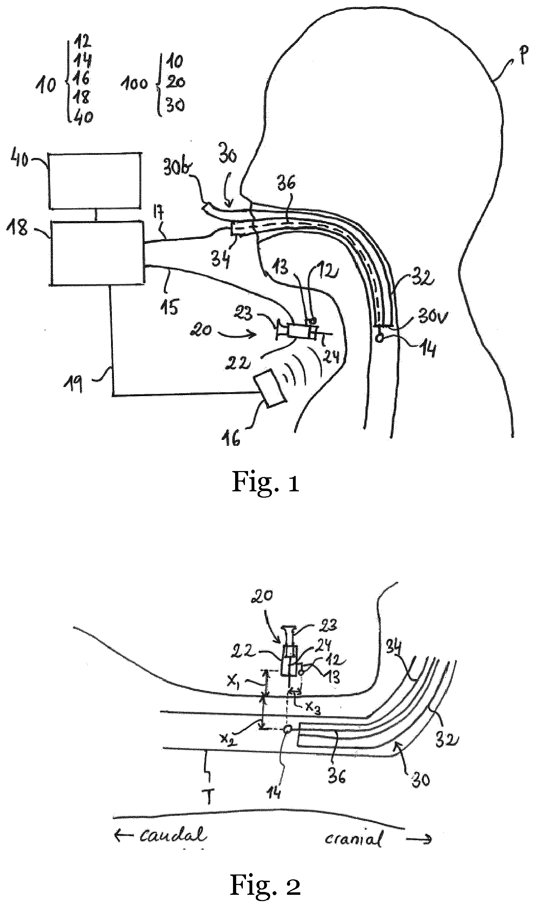

Fig. 1 shows a schematic illustration of a puncturing operation being performed in the neck region of a patient P as part of a PDT, for which a puncturing system 100 according to the invention is used. The puncturing system 100 comprises a monitoring system 10, a puncturing instrument 20 and an endoscopic instrument 30.

The monitoring system 10 comprises a first magnetic sensor 12, a second magnetic sensor 14, a field generator 16 and a processing unit 18. Further, in the embodiment shown, the monitoring system 10 comprises a graphical representation unit 40.

Each of the first and second magnetic sensors 12, 14 is configured in the exemplary embodiment shown in Fig. 1 as an electromagnetic inductive sensor comprising a plurality of induction coils. The field generator 16 is configured for generating a variable magnetic field, for example a magnetic field having a continuously variable magnetic field strength with a frequency of 800 Hz.

The first magnetic sensor 12 and the second magnetic sensor 14 are responsive to the magnetic field generated by the field generator 16. In the presence of the variable magnetic field generated by the field generator 16, the first magnetic sensor 12 generates first position data indicative of a position of the first magnetic sensor 12 in the variable magnetic field and first orientation data indicative of a 3d orientation of the first magnetic sensor 16 in the variable magnetic field. Further, the second magnetic sensor 14 reacts to the variable magnetic field generated by the field generator 16 by generating second position data indicative of a position of the second magnetic sensor 14 in the variable magnetic field. In some embodiments, the second magnetic sensor 14 can also be configured for additionally generating second orientation data indicative of an orientation of the second magnetic sensor 14 in space. The first position data, the first orientation data, and the second position data correspond to measurements of electromagnetically induced currents in the induction coils of the first magnetic sensor 12 and the second magnetic sensor 14, respectively. For example, the first position data, the first orientation data, and the second position data can be derived from measurements of current intensities corresponding to electromagnetically induced currents in the induction coils of the respective magnetic sensor induced by the variable magnetic field generated by the field generator 16.

The first position data and the first orientation data generated by the first magnetic sensor 12 as well as the second position data generated by the second magnetic sensor 14 are transmitted to the processing unit 18. In the exemplary embodiment shown in Fig. 1, the processing unit 18 is functionally connected to each of the first magnetic sensor 12 and the second magnetic sensor 14 by a corresponding wired connection 15 and 17, respectively. In the exemplary embodiment shown in Fig. 1, the processing unit 18 is also functionally connected to the field generator 16 by means of a corresponding wired connection 19.

The first magnetic sensor 12 is arranged on the puncturing instrument 20. In particular, in the exemplary embodiment shown in Fig. 1, the puncturing instrument 20 comprises a syringe 22 and an incision needle 24 that is movably received within the syringe 22, such that, the incision needle 24 is maximally received within the syringe 22 when a piston 23 of the syringe 22 is not actuated, and the incision needle 24 is pressed down and protrudes from the syringe 22 when the piston 23 of the syringe 22 is actuated. When the piston 23 is maximally compressed or actuated, the incision needle 24 maximally protrudes from the syringe 22, such that a distance between a tip of the incision needle 24 and the syringe 22 is maximal. In the exemplary embodiment shown in Fig. 1, the first magnetic sensor 12 is attached to the body of the syringe 22 (not to the movable incision needle 24) by means of an adapter 13 that rigidly connects the first magnetic sensor 12 to the syringe 22. Thus, when the incision needle 24 is moved with respect to the syringe by operating the piston 23, the position of the incision needle 24 with respect to the first magnetic sensor 12 varies, but the position of the syringe 22 with respect to the first magnetic sensor 12 does not vary.

The second magnetic sensor 14 is supported by the endoscopic instrument 30. In the exemplary embodiment shown in Fig. 1, the endoscopic instrument 30 is a bronchoscope comprising an optical channel 32 and a working channel 34 that runs in parallel to the optical channel 32. An optical fiber received within the optical channel 32 allows visual access to a region within the body of the patient P located in front of the visualisation tip 30v of the bronchoscope 30 from an observation and 30b of the bronchoscope 30 that is located opposite to the visualisation tip 30v. The working channel 34 is a hollow lumen of the bronchoscope 30 that allows additional instrumentation to be introduced into the area of the interior of the body of the patient P that is being observed through the optical channel 32. In the exemplary embodiment shown in Fig. 1, the second magnetic sensor 14 is supported by the bronchoscope 30 by means of a wire 36 that is received within the working channel 34 of the bronchoscope 30. The wire 36 provides structural support to the second magnetic sensor 14 and further provides a wired functional connection 17 between the second magnetic sensor 14 and the processing unit 18.

Since the first magnetic sensor 12 is attached to the puncturing instrument 20 and the second magnetic sensor 14 is supported by the endoscopic instrument 30, the first positional data provided by the first magnetic sensor 12 and the second positional data provided by the second magnetic sensor 14 can be used by the processing unit 18 to determine a relative position of the first magnetic sensor 12 with respect to the second magnetic sensor 14 based on the first and second position data and possibly based on information about the variable magnetic field obtained from the field generator 16 via the wired connection 19.

The processing unit 18 is configured for generating positional data in which the relative position of the first magnetic sensor 12 with respect to the second magnetic sensor 14 determined by the processing unit 18 is encoded. Further, the processing unit 18 can be configured for generating orientational data in which the relative orientation of the first magnetic sensor 12 with respect to the second magnetic sensor 14 determined by the processing unit 18 is encoded. The positional data and the orientational data can be provided in any suitable format.

The processing unit 18 is functionally connected to a graphical representation unit 40 and configured for outputting the generated positional data and orientational data to the graphical representation unit 40 in real-time. Using the positional data obtained from the processing unit 18, the graphical representation unit 40 can provide a graphical representation of the relative position and orientation of the puncturing instrument 20 with respect to the second magnetic sensor 14.

The position of the second magnetic sensor 14 within the trachea of the patient P can be visually monitored through the optical channel 32 of the bronchoscope 30 and can be adjusted, by correspondingly moving the wire 36 to which the second magnetic sensor 14 is attached, such that the second magnetic sensor 14 is arranged directly below the target puncturing location at which the skin of the patient should be punctured by the puncturing instrument 20.

Once the second magnetic sensor 14 is properly positioned within the trachea of the patient P for marking the position of the target puncturing location, the puncturing instrument 20 can be properly positioned and oriented in space, based on the assistance provided by the graphical representation of the positional data generated by the processing unit 18 in real-time, in order to puncture the skin of the patient precisely at the target puncturing location marked by the second magnetic sensor 14, and possibly with a target orientation of the puncturing instrument 20.

Fig. 2 shows a schematic zoomed-in view of the area of the neck of the patient P around the target puncturing location. In Fig. 2, the puncturing instrument 20 and the endoscopic instrument 30 are shown, but the processing unit 18 and any connections between the puncturing instrument 20 and the processing unit 18 as well as between the endoscopic instrument 30 and the processing unit 18 are omitted for illustrative simplicity.

As seen in Fig. 2, the first magnetic sensor 12 is attached to the main body of the syringe 22 by means of an adapter 13. When the piston 23 of the syringe 22 is actuated, the incision needle 24 is displaced down protruding from the syringe 22 towards the neck of the patient P and eventually reaching and puncturing the skin of the patient. The processing unit 18 is configured for taking into account a relative position and a relative orientation of the first magnetic sensor 12 with respect to the syringe 22 and or the incision needle 24, as determined by the adapter 13, in order to determine a relative position of the puncturing instrument 20 with respect to the second magnetic sensor 14 based on the first and second position data. For example, the processing unit 18 can be configured for taking into account a vertical offset x₁ indicated in Fig. 2 and corresponding to a distance in the ventral-dorsal direction between the first magnetic sensor 12 and a distal end or tip of the incision needle 24 when the incision needle 24 is maximally protruding from the syringe 22, i.e. when the piston 23 is maximally descended. Further, the processing unit 18 can be configured to take into account a horizontal offset x₃ indicated in Fig. 2 and corresponding to a distance in the caudal-cranial direction between the first magnetic sensor 12 and said distal end or tip of the incision needle 24 when the incision needle 24 is maximally protruding from the syringe 22.

The processing unit 18 can also be configured for taking into account a vertical offset x₂ indicated in Fig. 2 and corresponding to a distance in the ventral-dorsal direction between the second magnetic sensor 14 and the skin of the patient at the target puncturing location, in this case the frontal surface of the neck.

By properly taking into account the distances x₁, x₂ and x₃, the processing unit 18 can encode in the positional data transmitted to the graphical representation unit 40 a relative position of the puncturing instrument 20 with respect to the second magnetic sensor 14 and/or with respect to target puncturing location marked by the second magnetic sensor 14. In addition, by properly taking into account an orientation of the first magnetic sensor 12 with respect to the puncturing instrument 20, the processing unit 18 can encode in the orientational data transmitted to the graphical representation unit 40 a relative orientation of the puncturing instrument 20 with respect to the second magnetic sensor 14 and/or with respect to the target puncturing location marked by the second magnetic sensor 14.

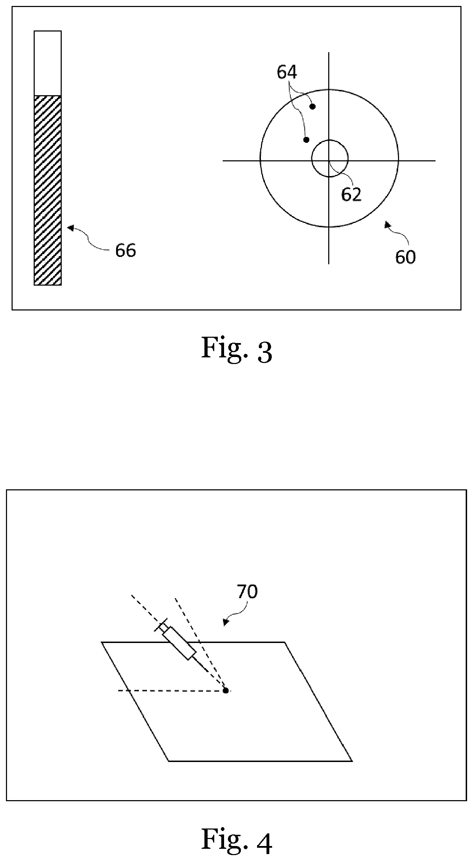

Fig. 3 shows a schematic illustration of a graphical representation 60 of the relative position of the puncturing instrument 20 with respect to the target puncturing location outputted by the graphical representation unit 40. In the exemplary embodiment shown, the processing unit 18 is configured for generating the positional data using a coordinate system in which the position of the second magnetic sensor 14, which corresponds to a position of the target puncturing location in the coronal plane, is used as an origin of coordinates.

In a schematic illustration of Fig. 3, the graphical representation 60 provided by the graphical representation unit 40 comprises a representation of a current position in the coronal plane of a point in space corresponding to a virtual position reachable by the tip of the incision needle 24 when the incision needle 24 maximally protrudes from the syringe 22 (which is derived by the processing unit 18 from a current position of the first magnetic sensor 12 as previously explained) with respect to a current position in the coronal plane of the second magnetic sensor 14, which is used as the origin of coordinates and corresponds to the centre 62 of the cross profile shown in the right-side graphical representation 60 illustrated in Fig. 3. The two dots 64 that are offset from the centre 62 of the cross profile illustrate different positions of the aforementioned virtual position reachable by the tip of the incision needle 24 corresponding to respective positions of the puncturing instrument 20 in space. When a dot 64 is represented coinciding with the centre of the cross profile, i.e. with the origin of coordinates 62, this indicates that the relative position of the puncturing instrument 20 with respect to the second magnetic sensor 14 (and hence with respect to the target puncturing location) in the coronal plane (perpendicular to the ventral-dorsal direction) is adequate for performing a puncturing operation at the target puncturing location.

The graphical representation provided by the graphical representation unit 40 based on the positional data generated by the processing unit 18 further comprises, as illustrated in the left-side graphical representation 66 illustrated in Fig. 3, an indication of a distance between the aforementioned virtual position reachable by the tip of the incision needle 24 when the incision needle 24 maximally protrudes from the syringe 22 and the second magnetic sensor 14 in the ventral-dorsal direction perpendicular to the coronal plane. The graphical representation 66 may assist a user of the puncturing system 100 deciding when a distance between the puncturing instrument 20 and the skin of the patient is appropriate for initiating the puncturing operation by pressing the piston 23 of the syringe 22.

The processing unit 18 can be configured for generating an indication signal when the relative position of the first magnetic sensor 12 with respect to the second magnetic sensor 14 determined by the processing unit 18 (or a corresponding relative position of the puncturing instrument 20 with respect to the target puncturing location and/or to the second magnetic sensor 14) corresponds to a target relative position within a predefined tolerance. The target relative position can be set such that, when the puncturing instrument 20 is in the target relative position, the position of the syringe 22 is such that the incision needle 24 can be used for puncturing the skin of the patient at the target puncturing location by simply actuating the piston 23 of the syringe 22, without moving the syringe, i.e. while keeping the syringe static, until the tip of the incision needle 24 reaches the skin of the patient. The indication signal can for example trigger a colour in the graphical representation 60 and/or an acoustic indicator, indicating to a user of the puncturing system 100, that the puncturing system 20 is properly positioned and/or oriented and that the puncturing operation can be performed.

Fig. 4 shows a further schematic illustration of a 3d graphical representation 70 of the relative orientation of the puncturing instrument 20 that can be graphically represented in real-time by the graphical representation unit 40 based on the positional data and the orientational data provided by the processing unit 18. Relying on information provided by the graphical representation 70, a user of the puncturing system 100, it can be a surgeon or an intensivist, can arrange the puncturing instrument 20 in a target orientation in order to reach the target puncturing location with the target orientation.

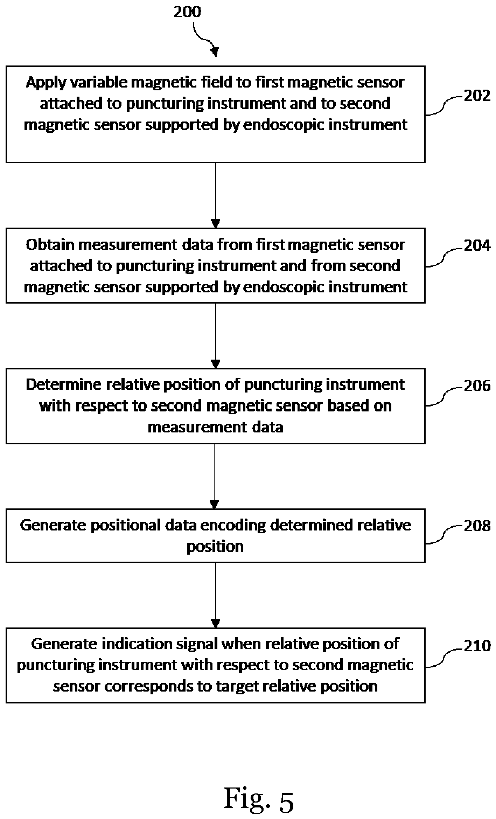

Fig. 5 is a flow diagram schematically illustrating a method 200 of generating positional data for monitoring a surgical puncturing operation according to the present invention. The method 200 can be carried out using the monitoring system 10 described with respect to Figs. 1 and 2, possibly as part of a puncturing system, for example the puncturing system 100 described above with respect to Figs. 1 and 2.

In step 202 of the method 200, a variable magnetic field is generated, for example by the field generator 18, and is sensed by the first magnetic sensor 12 and by the second magnetic sensor 14.

In step 204, first measurement data and second measurement data are obtained, in particular by the processing unit 18. The first measurement data can be obtained from the first magnetic sensor 12 and correspond to a position of the first magnetic sensor 12. Since the first magnetic sensor 12 is attached to the puncturing instrument 20, it is possible to derive a position of the puncturing instrument 20 from the first measurement data. The second measurement data can be obtained from the second magnetic sensor 14 and correspond to a position of the second magnetic sensor 14. Since the second magnetic sensor 14 is supported by the endoscopic instrument 30, for example by the wire 36, and a target puncturing location can be visualised using the endoscopic instrument 30, it is possible to derive a position of the target puncturing location from the second measurement data.

Optionally, the first measurement data obtained in step 204 can further correspond to an orientation of the first magnetic sensor 12 and/or to a corresponding orientation of the puncturing instrument 20.

In step 206, the first measurement data and the second measurement data are used for determining a relative position of the puncturing instrument 20 with respect to the second magnetic sensor 14 (and hence with respect to the target puncturing location).

Optionally, if the first measurement data obtained in step 204 also correspond to an orientation of the first magnetic sensor 12 and or to a corresponding orientation of the puncturing instrument 20, step 206 can comprise determining an orientation of the puncturing instrument 20 based on the first measurement data.

In step 208, positional data encoding the determined relative position are generated. The positional data can be generated using a coordinate system in which the position of the second magnetic sensor 14 corresponds to the origin of coordinates. Optionally, in step 208, orientational data encoding an orientation of the puncturing instrument 20 can be generated. The positional data and the optional orientational data can be outputted to a graphical representation unit like the graphical representation unit 40 of the monitoring system 10 described with respect to Figs. 1 and 2.

The method 200 can optionally comprise an additional step 210 of generating an indication signal when the relative position of the puncturing instrument 20 with respect to the second magnetic sensor 14 encoded in the positional data corresponds to a target relative position within a predefined tolerance, for example within a tolerance of 1 mm. The target relative position can for example be set such that, when the puncturing instrument 20 is in the target relative position, the position of the syringe 22 is such that the incision needle 24 can be used for puncturing the skin of the patient at the target puncturing location by simply actuating the piston 23 of the syringe 22, without moving the syringe, i.e. while keeping the syringe static, until the tip of the incision needle 24 reaches the skin of the patient.

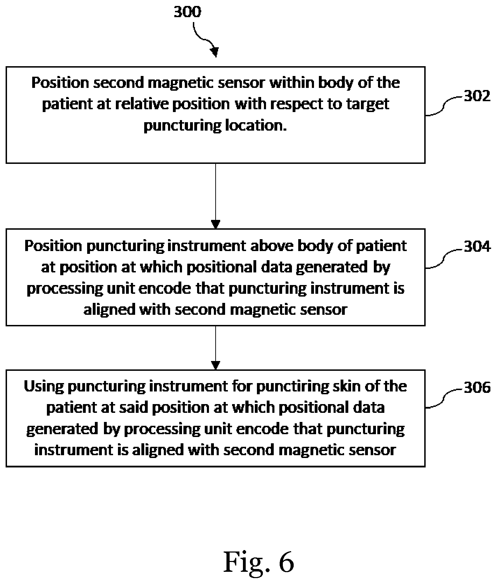

Fig. 6 is a flow diagram schematically illustrating a method 300 of performing a surgical puncturing operation using the puncturing system 100 described above with respect to Figs. 1 and 2. This surgical puncturing operation can be part of a PDT.

In step 302, the second magnetic sensor 14, which is supported by the endoscopic instrument 30, for example by means of the wire 36 (cf. Fig. 1 and 2), is positioned within the body of the patient P at a relative position with respect to a target puncturing location. For example, this can comprise positioning the second magnetic sensor 14 within the trachea of the patient P below the target puncturing location, for example through the working channel 34 of the endoscopic instrument 30 as illustrated in Figs. 1 and 2.

In step 304, the puncturing instrument 20, to which the first magnetic sensor 12 is attached, is positioned above the body of the patient at a position at which the positional data generated by the processing unit 18 encode that the position of the puncturing instrument 20, for example the aforementioned virtual position of the tape of the incision needle 24, corresponds to the position of the second magnetic sensor 14 (e.g. is aligned with the second magnetic sensor 14), for example in the coronal plane, as previously described. Step 304 can be carried out while monitoring the position and/or orientation of the puncturing instrument 20 in real time using a graphical representation of the positional data and/or orientational data provided by the processing unit 18 of the monitoring system 10 of the puncturing system 100.

In step 306, once the puncturing instrument 20 is properly positioned used the assistance provided by the positional data generated by the processing unit 18, the puncturing instrument 20 is used for puncturing the body of the patient at said position at which the positional data generated by the processing unit 18 reveal that the position of the puncturing instrument 20, for example the aforementioned virtual position of the tip of the incision needle 24, corresponds to the position of the second magnetic sensor 14, for example in the coronal plane, as previously described. This ensures that the puncturing instrument 20 punctures the body of the patient at the target puncturing location.

Optionally, if the first magnetic sensor 12 is configured for generating orientation data indicative of an orientation of the puncturing instrument 20 and the processing unit 18 is configured for determining an orientation of the puncturing instrument based thereon, step 304 can comprise, in addition to positioning the puncturing instrument 20, orienting the puncturing instrument 20 with a target orientation. Then, in step 306, the puncturing instrument 20 can be oriented with the target orientation when puncturing the body of the patient.

Although preferred exemplary embodiments are shown and specified in detail in the drawings and the preceding specification, these should be viewed as purely exemplary and not as limiting the invention. It is noted in this regard that only the preferred exemplary embodiments are shown and specified, and all variations and modifications should be protected that presently or in the future lie within the scope of protection of the invention as defined in the claims.

## Claims

1. A monitoring system for monitoring a surgical puncturing operation comprising:
a first magnetic sensor arrangeable on and/or attachable to a puncturing instrument (20) configured for performing a surgical puncturing operation;
a second magnetic sensor (14) supportable by an endoscopic instrument (30);
a field generator (16) configured for generating a variable magnetic field;
wherein the first magnetic sensor (12) is configured for generating first position data in response to the magnetic field generated by the field generator (16) indicative of a position of the first magnetic sensor (12), and wherein the second magnetic sensor (14) is configured for generating second position data in response to the magnetic field generated by the field generator (16) indicative of a position of the second magnetic sensor (14); and
a processing unit (18) configured for determining a relative position of the first magnetic sensor (12) with respect to the second magnetic sensor (14) based on the first and second position data;
wherein the processing unit (18) is further configured for generating positional data encoding the determined relative position of the first magnetic sensor (12) with respect to the second magnetic sensor (14) and optionally for outputting the generated positional data to a graphical representation unit, preferably in real time.

2. The system of claim 1, wherein the positional data is based on a coordinate system having the position of the second magnetic sensor (14) as an origin of coordinates.

3. The system of claim 1 or 2, further comprising a graphical representation unit (40) configured for providing a graphical representation of the relative position of the first magnetic sensor (12) with respect to the second magnetic sensor (14), preferably in real time.

4. The system of any of the preceding claims, wherein the processing unit (18) is further configured for generating an indication signal when the determined relative position corresponds to a target relative position within a predefined tolerance.

5. The system of any of the preceding claims, wherein the first magnetic sensor (12) is further configured for generating first orientation data in response to the magnetic field generated by the field generator (16), wherein the first orientation data are indicative of an orientation of the first magnetic sensor (12) and/or of the puncturing instrument (20), preferably with respect to the second magnetic sensor (14), and
wherein the processing unit (18) is further configured for determining an orientation of the first magnetic sensor (12) and/or of the puncturing instrument (20), preferably with respect to the second magnetic sensor (14), based on the first orientation data.

6. The system of claim 5, wherein the processing unit (18) is further configured for generating orientational data encoding the orientation of the first magnetic sensor (12) and/or of the puncturing instrument (20), preferably with respect to the second magnetic sensor (14), and optionally for outputting the generated orientational data to a graphical representation unit (40), preferably in real time.

7. The system of claim 5 or 6, further comprising a second graphical representation unit configured for providing a graphical representation of the orientation of the first magnetic sensor (12) and/or of the puncturing instrument (20), preferably with respect to the second magnetic sensor (14), preferably in real time.

8. The system of any of the preceding claims, wherein the processing unit (18) is connected with the first magnetic sensor (12) and/or the second magnetic sensor (14) via a wired connection.

9. The system of any of the preceding claims, further comprising an adapter (13) for arranging the first magnetic sensor (12) on a puncturing instrument (20) at a fixed position and/or at a fixed orientation.

10. A puncturing system (100) for performing a surgical puncturing operation comprising:
a monitoring system according to any of claims 1 to 9;
a puncturing instrument (20) configured for performing a surgical puncturing operation on the body of a patient, wherein the first magnetic sensor (12) of the monitoring system is arrangeable or arranged on the puncturing instrument (20); and
an endoscopic instrument (30) configured for providing endoscopic monitoring of the surgical puncturing operation by the puncturing instrument (20), in particular from within the body of the patient, wherein the second magnetic sensor (14) of the monitoring system is supportable by the endoscopic instrument (30);
wherein the processing unit (18) of the monitoring system (10) is preferably configured for determining a relative position of the puncturing instrument (20) with respect to the second magnetic sensor (14) based on the first and second position data; and
wherein the processing unit (18) is preferably further configured for generating the positional data encoding the determined relative position of the puncturing instrument (20) with respect to the second magnetic sensor (14).

11. A method of generating positional data for monitoring a surgical puncturing operation, the method comprising:
obtaining first measurement data corresponding to a position of a first magnetic sensor (12) arranged on and/or attached to a puncturing instrument (20) configured for performing a surgical puncturing operation on the body of a patient;
obtaining second measurement data corresponding to a position of a second magnetic sensor (14) supported by an endoscopic instrument (30) configured for providing endoscopic monitoring of the surgical puncturing operation by the puncturing instrument (20) from within the body of the patient;
wherein the first and second measurement data are respectively obtained in response to a variable magnetic field to which the first and second magnetic sensors (12, 14) are exposed; and
determining a relative position of the first magnetic sensor (12) and/or of the puncturing instrument (20) with respect to the second magnetic sensor (14) based on the first and
second measurement data; and
generating positional data encoding the determined relative position.

12. The method of claim 11, further comprising generating a indication signal when the determined relative position corresponds to a target relative position within a predefined tolerance.

13. The method of claim 11 or 12, further comprising outputting the generated positional data to a graphical representation unit (40); and/or
wherein the positional data is generated based on a coordinate system having the position of the second magnetic sensor (14) as an origin of coordinates.

14. The method of any of claims 11 to 13, wherein the first measurement data further correspond to an orientation of the first magnetic sensor (12) and/or of the puncturing instrument (20);
wherein the method further comprises determining an orientation of the first magnetic sensor (12) and/or of the puncturing instrument (20) based on the first measurement data and generating orientational data encoding the determined orientation of the first magnetic sensor (12) and/or of the puncturing instrument (20);
wherein the method preferably further comprises outputting the generated orientational data to a graphical representation unit (40).

15. A memory device storing executable instructions which, when executed by a processor, cause the processor to perform the method of any of claims 11 to 14.
